# EUROPEAN PATENT SPECIFICATION

(11) **EP 2 032 988 B1**
(45) Date of publication and mention of the grant of the patent: **08.12.2010**
(21) Application number: 07733395.3
(22) Date of filing: 28.06.2007
(51) Int. Cl.: G01N 33/569, G01N 33/58, G01N 33/542

(54) **MULTIPLE LABELLING FOR ANALYTE DETECTION**
MEHRFACHMARKIERUNG FÜR DEN NACHWEIS VON ANALYTEN
MARQUAGE MULTIPLE POUR DETECTION ANALYTIQUE

(30) Priority: 28.06.2006 GB 0612825
(43) Date of publication of application: 11.03.2009
(73) Proprietor: ITI Scotland Limited, 191 West George Street Glasgow G2 2LB (GB)
(72) Inventor: BLAIR, Edward, Caxton, Cambs, CB3 8PQ (GB)
(74) Representative: Hill, Christopher Michael
(86) International application number: PCT/GB2007/002404
(87) International publication number: WO 2008/001084

(56) References cited:
- DE-A1- 10 132 405
- US-A1- 2004 002 089
- GOWEN BRENT ET AL: "The tailless icosahedral membrane virus PRD1 localizes the proteins involved in genome packaging and injection at a unique vertex." JOURNAL OF VIROLOGY, vol. 77, no. 14, July 2003 (2003-07), pages 7863-7871, XP002453322 ISSN: 0022-538X
- SCHMIDTKE MICHAELA ET AL: "Susceptibility of coxsackievirus B3 laboratory strains and clinical isolates to the capsid function inhibitor pleconaril: antiviral studies with virus chimeras demonstrate the crucial role of amino acid 1092 in treatment" JOURNAL OF ANTIMICROBIAL CHEMOTHERAPY, vol. 56, no. 4, October 2005 (2005-10), pages 648-656, XP002453323 ISSN: 0305-7453

## Description

The present invention is concerned with improved methods of detecting analytes, in particular methods that are capable of detecting viral analytes, such as whole viruses, viral particles and viral components. The method is particularly advantageous, since it is capable of detecting analytes without resorting to complex and costly amplification techniques, such as PCR.

It is a well known method to detect pathogens, such as viruses, by reacting a component of the pathogen that is characteristic of the pathogen with a label. The label may then be detected in order to determine the presence, or absence of the pathogen. In some methods, the quantity of the label may provide further information about the quantity of the pathogen. Examples of this approach include detection of nucleic acid using nucleic acid probes that are complementary to specific genes or sequences in the pathogen. Typical methods also involve the use of antibodies capable of binding to particular antigenic regions of the pathogen (such as surface proteins in a virus) to identify the specific pathogen.

In some methods, a plurality of analyte pathogens can be detected in the same procedure, by using multiplexing techniques. These techniques typically involve the use of two or more different labels which are designed to be specific for two or more specific pathogens. These labels will be individually identifiable (e.g. by possessing different fluorescent groups) so that they can be related to the different analytes even if they are present in the same reaction zone.

However, all of these methods have a significant problem in that they suffer from a lack of sensitivity, particularly if the pathogens are present in low concentration. In the past, attempts have been made to solve this problem by concentrating the sample so as to increase the available quantity of pathogen, per unit volume of sample, or (in the case of nucleic acid in particular) by amplifying the pathogen component in the sample. Although both of these techniques can lead to greater sensitivity of detection, they have the significant drawback that they greatly increase the complexity of the method. Such a method requires much more sophisticated sample preparation, requires many more reagents and needs further reaction or sample preparation zones in the apparatus. There is also a requirement to remove waste from the concentrating and/or amplification procedure. All of this increases the cost of the method and the apparatus used to carry out the method. Furthermore, although sensitivity is increased, the extra method steps introduce the possibility that additional systemic errors exist in the final data, which in turn will require additional data processing to eliminate, further increasing complexity and cost.

Accordingly, there is a requirement for a more simple and cost effective solution to increasing the sensitivity of diagnostic methods and pathogen detection methods.

Gowen et al.2003 (Journal of Virology, volume 77, no.14, page 7863-7871) teaches the use of immunolabelling to localise proteins on the surface of the virus PRD1.

DE 10132405 teaches the detection of multimerisation of viral structural proteins comprising contacting a protein with a binding nucleic acid and then monitoring the change in fluorescence.

Schmidtke et al. 2005 (Journal of Antimicrobial Chemotherapy, volume 56, page 648-656) teaches the susceptibility of coxsackievirus B3 laboratory strains and clinical isolates to the capsid function inhibitor pleconaril.

US2004/0002089 teaches methods for sensitively detecting proximity changes in systems that utilize an interacting fluorophore and quencher.

It is an aim of the present invention to solve the problems associated with the known methods described above. In particular, it is an aim of the present invention to provide uses and methods of detection of analytes, and methods of diagnosis of target analytes or pathogens, which have good sensitivity, but are more simple and cost effective than known sensitive methods.

Accordingly, the present invention provides a method of diagnosing the presence of a pathogen in a subject, which method comprises:
(a) providing a sample obtained from the subject;
(b) detecting the absence or the presence and/or quantity of a pathogen protein in the sample by:
   (i) contacting the sample with a label capable of binding to the pathogen;
   (ii) detecting the presence and/or quantity of the label bound to the pathogen; and
   (iii) thereby detecting the presence and/or quantity of the pathogen in the sample;
   and
(c) making a diagnosis of the subject based on the absence or the presence and/or quantity of the pathogen,
wherein, the pathogen comprises a repeating protein unit, and wherein a plurality of label entities are capable of binding to a single pathogen entity such that a signal obtained for a pathogen from a plurality of labels is stronger than the signal obtained for a pathogen with a single label.

The present method has the advantage that a single entity of the analyte binds to more than one label entity. Thus, a single analyte entity will give rise to a much stronger signal (e.g. a higher amplitude of emission if fluorescence detection is employed) and sensitivity is increased. In the case of a whole virus, the protein coat is largely comprised of repeating geometric structures based on several n-fold (3-, 4-, 5-, 6-fold) axes of symmetry. Multiple labelling entities can attach to the protein capsid (up to several hundred without steric hindrance in some cases) and many times the normal sensitivity can therefore be achieved. The ability of a plurality of similar or identical entities to bind to a viral protein coat (capsid) has been verified by modelling in the human rhinovirus system (Steindl TM, Crump CE, Hayden FG, Langer T, J. Med. Chem. 2005, Oct. 6; 48(20), pp6250-60 "Pharmacophore modelling, docking, and principal component analysis based clustering: combined computer-assisted approaches to identify new inhibitors of the human rhinovirus coat protein". Published international patent application WO 03/068222 discloses methods of reducing rhinovirus contagion and related compositions. Ryan J et al disclose a new oral rhinovirus inhibitor BTA798 in Antiviral Research 2005, 65(3) (Abs LB11). However, these disclosures deal principally with antiviral methods for treatment of disease. The ability of certain species to bind in the above-mentioned way has never before been utilised to improve sensitivity in detection and diagnosis methods.

The present invention further provides a method for detecting an analyte in a sample, which method comprises:
(a) contacting the sample with a label capable of binding to the analyte;
(b) detecting the presence and/or quantity of the label bound to the analyte; and
(c) thereby detecting the presence and/or quantity of the analyte in the sample;
wherein, the analyte comprises a repeating protein unit, and wherein a plurality of label entities are capable of binding to a single analyte entity such that a signal obtained for an analyte from a plurality of labels is stronger than the signal obtained for an analyte with a single label, and wherein the label is a capsid function inhibitor.

Preferably, the analyte comprises a repeating protein unit of a virus selected from adenoviruses, herpesviruses, rotaviruses, reoviruses, picornaviruses, papillomaviruses, polyomaviruses, parvoviruses, parvoviruses, influenza viruses, flaviviruses and retroviruses.

Also provided by the present invention is use of a label in a method for detecting an analyte in a sample, for increasing the sensitivity of detection of the analyte, wherein, the analyte comprises a repeating protein unit, and wherein a plurality of label entities are capable of binding to a single analyte entity such that a signal obtained for an analyte from a plurality of labels is stronger than the signal obtained for an analyte with a single label, wherein the label comprises a capsid function inhibitor.

The invention will be described in more detail in the following, by way of example only, with reference to the following Figures, in which:

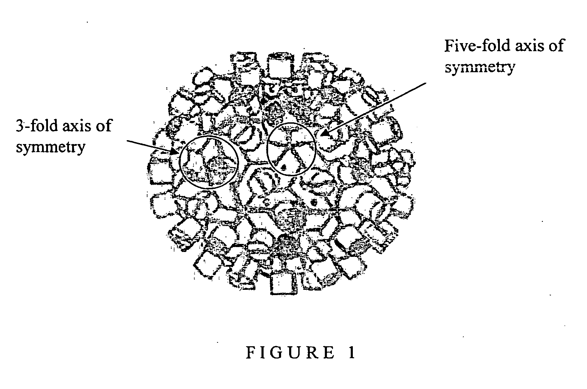

Figure 1 shows a symmetrical repeating unit of a typical virus particle;
Figure 2 shows the binding of a small molecule entity to the surface of a virus particle; and
Figure 3 shows a molecular beacon binding to a virus particle and emissions detected from it.

In the present invention, the analyte comprises a repeating protein unit. The repeating unit is required in order that more than one (preferably many) label entities may bind to the analyte. However, the type of protein is not especially limited provided that there are repeating units. Thus, the analyte may comprise a protein selected from a virus protein, a bacterial protein, and a mammalian protein (such as a human protein).

In some preferred embodiments of the present invention, the analyte may comprise a virus protein. In these embodiments, the analyte may comprise the whole of, or a portion of, a viral surface protein. However, the analyte is not otherwise especially limited, provided that a plurality of label entities are capable of binding to it. Thus, the analyte may be selected from, whole virus, viral particles, sub-viral particles, immature viral particles, and proto-viral complexes. The virus may be any type of virus, such as a bacteriophage.

In the method of diagnosis of the present invention the label itself is not especially limited, provided that a plurality of labelling entities can attach to a single analyte entity. The type of label employed may be selected depending on the analyte under investigation. Thus, for a whole virus, a larger label might be employed than would be for a single viral coat protein molecule, due to steric constraints. The label may comprise an antibody, a peptide, an aptamer, a nucleic acid, or an organic compound or molecule. In one embodiment of the method of diagnosis, and in the method for detecting an analyte and use of a label according to the present invention, the label comprises a capsid function inhibitor. In this embodiment, the label more preferably comprises an oxadiazole compound. The most preferred such compound is Pleconaril or a Pleconaril derivative.

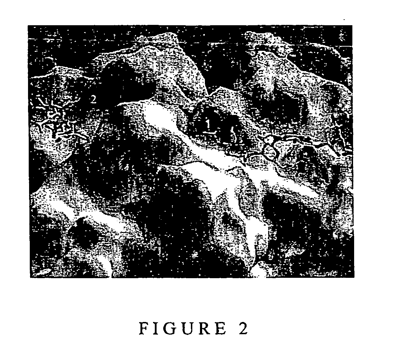

Typically the label comprises a reporter group, such as a fluorescent group that is excited at a wavelength differing from its emission wavelength, Preferably, the reporter group is altered when the label binds to the analyte, as shown in Figure 2, where the unbound entity 2 undergoes a conformational change to give the extended form of entity 1. In some preferred embodiments, the reporter group comprises a fluorescent group that fluoresces differently in its extended form. In these embodiments, typically the label is internally quenched such that binding to an analyte (such as a virus particle) ablates quenching and leads to detectable signal output.

Preferably the pathogen is selected from a virus and a bacterium. The invention may apply to any virus. Preferred viruses include adenoviruses, herpesviruses, rotaviruses, reoviruses, picornaviruses, papillomaviruses, polyomaviruses, parvoviruses, parvoviruses, influenza viruses, flaviviruses (such as HCV) and retroviruses (such as HIV). Also included are animal viruses, plant viruses and bacterial viruses (bacteriophages), including Lambda, PhiX174, PRD1, MS2, and T-even.

In the most preferred embodiments, the virus is selected from an HCV, HIV, herpes simplex virus, human rhinovirus, influenza virus, and a viral pathogen of a non-human species.

In this method the subject is typically a mammal, and is preferably human.

As has been mentioned briefly above, it is a preferred aspect of the present invention that cooperative binding is employed in putting the invention into effect, in particular so as to make the most of the signal enhancement achieved with the present uses and methods. Such binding is typically termed 'molecular beacon' technology.

There are two aspects to molecular beacons, both of which are applicable to the present invention. Firstly, cooperative binding and secondly, cooperative signal amplification. Co-operative binding is often seen in nature where the binding of one ligand promotes the binding of another to the same target macromolecule. The best known example is oxygen saturation of haemoglobin which has a "sigmoidal" binding curve. The binding of proteins to nucleic acid, e.g., the lac repressor and the lac operator, are also known. The impact in the present invention is that co-operativity will detect even small quantities of target because the small molecule ligand will be in excess.

Co-operative signalling is also easily employed in the invention. The binding of one tagged ligand in proximity to another is sufficient to enhance the signal released by both tags; it is an inverse situation to signal quenching systems, which have been described above and are also well known. It may mean that the detection ligands consist of two or more different tags that can either be resident on the same ligand (like an inverse molecular beacon; see http://www.molecular-beacons.org/) or may be on separate ligands.

The invention will now be described in further detail by way of example only, with reference to the following specific embodiments.

### EXAMPLES

In one specific embodiment, virus containing body fluids, such as whole blood, plasma or serum, or urine, or cerebro-spinal fluid are reacted with a specified quantity of internally quenched detection agent.

After an incubation period, a light of specified wavelength (excitation wavelength) is shone on the reacted fluids and fluorescent emissions at a pre-determined wavelength are measured. The level of light emission is proportional to the bound fluorophore and no signal is given from unbound, and therefore quenched, fluorophore.

Although binding of single fluorescent molecule to each virus particle will give a signal, the virus particle is able to bind a multiplicity of the fluorescent molecules and thus a secondary level of signal amplification is given.

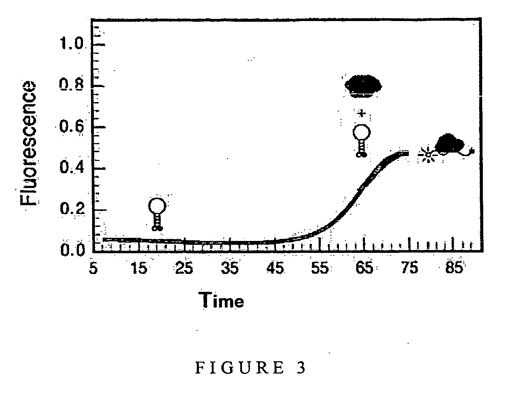

An example of molecular beacon binding to a virus particle and emissions detected from it is shown in Figure 3. In the present embodiment, a similar plot of emission detection reveals the presence or absence of the virus particle. The intensity of the peak in the spectrum provides further quantitative information.

## Claims

1. A method of diagnosing the presence of a pathogen in a subject, which method comprises:
(a) providing a sample obtained from the subject;
(b) detecting the absence or the presence and/or quantity of a pathogen protein in the sample by:
(i) contacting the sample with a label capable of binding to the pathogen;
(ii) detecting the presence and/or quantity of the label bound to the pathogen; and
(iii) thereby detecting the presence and/or quantity of the pathogen in the sample;
and
(c) making a diagnosis of the subject based on the absence or the presence and/or quantity of the pathogen,
wherein, the pathogen comprises a repeating protein unit, and wherein a plurality of label entities are capable of binding to a single pathogen entity such that a signal obtained for a pathogen from a plurality of labels is stronger than the signal obtained for a pathogen with a single label.

2. A method according to claim 1, wherein the pathogen is selected from a virus and a bacterium, preferably wherein the virus is selected from an HCV, HIV, herpes simplex virus, human rhinovirus, influenza virus, and a viral pathogen of a non-human species, and/or preferably wherein the subject is a mammal, preferably a human.

3. A method according to claim 1 or claim 2, wherein the pathogen is selected from, whole virus, viral particles, viral protein, sub-viral particles, immature viral particles, and proto-viral complexes.

4. A method according to any preceding claim, wherein the label comprises an antibody, an aptamer, a nucleic acid, or an organic compound or molecule, and preferably wherein the label comprises a capsid function inhibitor, and/or preferably wherein the label comprises an oxadiazole compound, preferably Pleconaril or a Pleconaril derivative.

5. A method according to any preceding claim, wherein the label comprises a reporter group, preferably wherein a property of the reporter group is altered when the label binds to the pathogen, and/or preferably wherein the reporter group comprises a fluorescent group.

6. A method according to any preceding claim, wherein the signal is generated using a molecular beacon.

7. Use of a label in a method for detecting an analyte in a sample, for increasing the sensitivity of detection of the analyte, wherein, the analyte comprises a repeating protein unit, and wherein a plurality of label entities are capable of binding to a single analyte entity such that a signal obtained for an analyte from a plurality of labels is stronger than the signal obtained for an analyte with a single label, wherein the label comprises a capsid function inhibitor.

8. Use according to claim 7, wherein the method comprises:
(a) contacting the sample with the label;
(b) detecting the presence and/or quantity of the label bound to the analyte; and
(c) thereby detecting the presence and/or quantity of the analyte in the sample.

9. Use according to claim 7 or claim 8, wherein the analyte is selected from, whole virus, viral particles, viral protein, sub-viral particles, immature viral particles, proto-viral complexes, HCV, HIV, herpes simplex virus, human rhinovirus, influenza, or a viral pathogen of non-human species.

10. Use according to any one of claims 7 to 9 wherein the label comprises an oxadiazole compound, preferably Pleconaril or a Pleconaril derivative.

11. Use according to any one of claims 7 to 10, wherein the label comprises a reporter group, preferably wherein a property of the reporter group is altered when the label binds to the analyte, and/or preferably wherein the reporter group comprises a fluorescent group.

12. Use according to any one of claims 7 to 11, wherein the signal is generated using a molecular beacon.

13. A method for detecting an analyte in a sample, which method comprises:
(a) contacting the sample with a label capable of binding to the analyte;
(b) detecting the presence and/or quantity of the label bound to the analyte; and
(c) thereby detecting the presence and/or quantity of the analyte in the sample;
wherein, the analyte comprises a repeating protein unit, and wherein a plurality of label entities are capable of binding to a single analyte entity such that a signal obtained for an analyte from a plurality of labels is stronger than the signal obtained for an analyte with a single label, and wherein the label is a capsid function inhibitor.

14. A method according to claim 13, wherein the analyte comprises a repeating protein unit of a virus selected from adenoviruses, herpesviruses, rotaviruses, reoviruses, picornaviruses, papillomaviruses, polyomaviruses, parvoviruses, parvoviruses, influenza viruses, flaviviruses and retroviruses, preferably wherein the virus is selected from HCV, HIV, or herpes simplex virus, human rhinovirus, influenza, or viral pathogen of non-human species.

15. A method according to claim 13 or claim 14, which method is a method as further defined in claims 9 to 12.

## Patentansprüche

1. Ein Verfahren zur Diagnose der Präsenz eines Pathogens in einem Subjekt, wobei das Verfahren folgende Schritte aufweist:
(a) Bereitstellen einer Probe, die von dem Subjekt erhalten wurde;
(b) Erfassen der Abwesenheit oder der Präsenz und/oder Menge eines pathogenen Proteins in der Probe durch:
(i) Kontaktieren der Probe mit einer Markierung, die in der Lage ist, sich an das Pathogen zu binden;
(ii) Nachweis der Präsenz und/oder Menge der Markierung, die an das Pathogen gebunden ist; und
(iii) hierdurch Nachweis der Präsenz und/oder Menge des Pathogen in der Probe; und
(c) Durchführen einer Diagnose des Subjekts auf der Basis der Abwesenheit oder der Präsenz und/oder Menge des Pathogens,
wobei das Pathogen eine wiederholende Proteineinheit aufweist und wobei eine Mehrzahl von Markierungseinheiten in der Lage ist, sich an eine einzelne Pathogeneinheit zu binden, so dass ein Signal, dass von mehreren Markierungen für das Pathogen erhalten wird, stärker ist als das Signal, das mit einer einzigen Markierung für ein Pathogen erhalten wird.

2. Ein Verfahren nach Anspruch 1, wobei das Pathogen ausgewählt wird aus einem Virus und einem Bakterium, wobei das Virus vorzugsweise aus HCV, HIV, Herpes simplex Virus, menschlichem Rhinovirus, Influenzavirus und einem viralen Pathogen einer nicht menschlichen Spezies ausgewählt ist und/oder wobei das Subjekt vorzugsweise ein Säugetier, vorzugsweise ein Mensch ist.

3. Ein Verfahren nach Anspruch 1 oder Anspruch 2, wobei das Pathogen ausgewählt wird aus einem ganzem Virus, viralen Partikeln, viralen Porteinen, subviralen Partikeln, unreifen viralen Partikeln und protoviralen Komplexen.

4. Ein Verfahren nach einem der vorhergehenden Ansprüche, wobei die Markierung einen Antikörper, ein Aptamer, eine Nukleinsäure oder eine organische Verbindung oder Molekül aufweist, und wobei die Markierung vorzugsweise einen Capsid-Funktionsinhibitor aufweist, und/oder wobei die Markierung vorzugsweise eine Oxodiazolverbindung, vorzugsweise Pleconaril oder ein Pleconarilderivat aufweist.

5. Ein Verfahren nach einem der vorherigen Ansprüche, wobei die Markierung einer Reportergruppe aufweist, wobei vorzugsweise eine Eigenschaft der Reportergruppe geändert wird, wenn sich die Markierung an das Pathogen bindet, und/oder wobei die Reportergruppe vorzugsweise eine fluoreszierende Gruppe aufweist.

6. Ein Verfahren nach einem der vorherigen Ansprüche, wobei das Signal mit Hilfe eines molekularen Leuchtfeuers generiert wird.

7. Verwendung einer Markierung in einem Verfahren zum Nachweis eines Analyten in einer Probe, zur Erhöhung der Sensitivität des Nachweises des Analyten, wobei der Analyt eine wiederholende Proteineinheit aufweist und wobei eine Mehrzahl von Markierungseinheiten in der Lage ist, sich an eine einzelne Analyteinheit zu binden, so dass ein Signal, das für einen Analyten von mehreren Markierungen erhalten wird, stärker ist als das Signal, das für einen Analyten mit einer einzigen Markierung erhalten wird, wobei die Markierung einen Capsid-Funktionsinhibitor aufweist.

8. Verwendung nach Anspruch 7, wobei das Verfahren folgende Schritte aufweist:
(a) Kontaktieren der Probe mit der Markierung;
(b) Nachweis der Präsenz und/oder Menge der an den Analyten gebundenen Markierung; und
(c) **dadurch** Nachweis der Präsenz und/oder Menge des Analyten in der Probe.

9. Verwendung nach Anspruch 7 oder Anspruch 8, wobei der Analyt ausgewählt wird aus einem vollständigen Virus, viralen Partikeln, viralem Protein, subviralen Partikeln, unreifen viralen Partikeln, protoviralen Komplexen, HCV, HIV, Herpes simplex Virus, humanem Rhinovirus, Influenza oder einem viralen Pathogen einer nicht menschlichen Spezies.

10. Verwendung nach einem der Ansprüche 7 bis 9, wobei die Markierung eine Oxodiazolverbindung, vorzugsweise Pleconaril oder ein Pleconarilderivat aufweist.

11. Verwendung nach einem der Ansprüche 7 bis 10, wobei die Markierung einer Reportergruppe aufweist, wobei vorzugsweise eine Eigenschaft der Reportergruppe geändert wird, wenn sich die Markierung an den Analyten bindet, und/oder wobei die Reportergruppe vorzugsweise eine fluoreszierende Gruppe aufweist.

12. Verwendung nach einem der Ansprüche 7 bis 11, wobei das Signal mit Hilfe eines molekularen Leuchtfeuers generiert wird.

13. Ein Verfahren für den Nachweis eines Analyten in einer Probe, wobei das Verfahren folgende Schritte aufweist:
(a) Kontaktieren der Probe mit einer Markierung, die in der Lage ist, sich an den Analyten zu binden;
(b) Nachweis der Präsenz und/oder Menge der an den Analyten gebundenen Markierung; und
(c) **dadurch** Nachweis der Präsenz und/oder Menge des Analyten in der Probe;
wobei der Analyt eine wiederholende Proteineinheit aufweist und wobei eine Mehrzahl von Markierungseinheiten in der Lage ist, sich an eine einzelne Analyteneinheit zu binden, so dass ein Signal, das von mehreren Markierungen für einen Analyten erhalten wird, stärker ist als das Signal, das für einen Analyten mit einer einzigen Markierung erhalten wird, und wobei die Markierung ein Capsid-Funktionsinhibitor ist.

14. Ein Verfahren nach Anspruch 13, wobei der Analyt eine wiederholende Proteineinheit eines Virus aufweist, der ausgewählt ist aus Adenoviren, Herpesviren, Rotaviren, Reoviren, Picornaviren, Papillomaviren, Polyomaviren, Parvoviren, Influenzaviren, Flaviviren und Retroviren, wobei das Virus vorzugsweise ausgewählt ist aus HCV, HIV oder Herpes simplex Virus, humanem Rhinovirus, Influenza oder viralem Pathogen einer nicht menschlichen Spezies.

15. Ein Verfahren nach Anspruch 13 oder Anspruch 14, wobei das Verfahren ein Verfahren ist, wie es in den Ansprüchen 9 bis 12 näher definiert ist.

## Revendications

1. Méthode de diagnostic de la présence d'un pathogène chez un sujet, ladite méthode comprenant :
(a) la fourniture d'un échantillon prélevé sur un sujet ;
(b) la détection de l'absence ou de la présence et/ou de la quantité d'une protéine pathogène dans l'échantillon en :
(i) mettant en contact l'échantillon avec un marqueur capable de se lier au pathogène,
(ii) détectant la présence et/ou la quantité du marqueur lié au pathogène ; et
(iii) détectant ainsi la présence et/ou la quantité du pathogène dans l'échantillon ;
et
(c) la réalisation du diagnostic du sujet, sur la base de l'absence ou de la présence et/ou de la quantité du pathogène,
dans laquelle le pathogène comprend une unité de protéine de répétition, et dans laquelle une pluralité d'entités de marqueurs est capable de se lier à une entité de pathogène unique, de sorte qu'un signal obtenu pour un pathogène provenant d'une pluralité de marqueurs soit plus fort que le signal obtenu pour un pathogène avec un marqueur unique.

2. Méthode selon la revendication 1, dans laquelle le pathogène est choisi à partir d'un virus et d'une bactérie, de préférence dans laquelle le virus est choisi parmi un HCV, HIV, le virus de l'herpes simplex, un rhinovirus humain, le virus de la grippe, et un pathogène viral d'une espèce non-humaine, et/ou de préférence dans laquelle le sujet est un mammifère, de préférence un être humain.

3. Méthode selon la revendication 1 ou 2, dans laquelle le pathogène est choisi parmi un virus complet, des particules virales, une protéine virale, des particules sous-virales, des particules virales immatures, et des complexes proto-viraux.

4. Méthode selon l'une quelconque des revendications précédentes, dans laquelle le marqueur comprend un anticorps, un aptamère, un acide nucléique, ou un composé ou molécule organique, et de préférence dans laquelle le marqueur comprend un inhibiteur de fonction capside, et/ou de préférence dans laquelle le marqueur comprend un composé oxadiazole, de préférence le Pléconaril ou un dérivé de Pléconaril.

5. Méthode selon l'une quelconque des revendications précédentes, dans laquelle le marqueur comprend un groupe rapporteur, de préférence dans laquelle une propriété du groupe rapporteur est altérée quand le marqueur se lie au pathogène et/ou de préférence dans laquelle le groupe rapporteur comprend un groupe fluorescent.

6. Méthode selon l'une quelconque des revendications précédentes, dans laquelle le signal est généré en utilisant une balise moléculaire.

7. Utilisation d'un marqueur dans une méthode de détection d'un analyte dans un échantillon, pour augmenter la sensibilité de détection de l'analyte, dans laquelle l'analyte comprend une unité de protéine de répétition, et dans laquelle une pluralité d'entités de marqueur est capable de se lier à une entité d'analyte unique, de sorte qu'un signal obtenu pour un analyte provenant d'une pluralité de marqueurs soit plus fort que le signal obtenu pour un analyte avec un marqueur unique, dans laquelle le marqueur comprend un inhibiteur de fonction capside.

8. Utilisation selon la revendication 7, dans laquelle la méthode comprend :
(a) la mise en contact de l'échantillon avec le marqueur,
(b) la détection de la présence et/ou de la quantité du marqueur lié à l'analyte ; et
(c) la détection de la présence et/ou de la quantité de l'analyte dans l'échantillon.

9. Utilisation selon la revendication 7 ou 8, dans laquelle l'analyte est choisi parmi un virus complet, des particules virales, une protéine virale, des particules sous-virales, des particules virales immatures, des complexes proto-viraux, le HCV, HIV, le virus de l'herpès simplex, le rhinovirus humain, la grippe ou un pathogène viral d'une espèce non-humaine.

10. Utilisation selon l'une quelconque des revendications 7 à 9, dans laquelle le marqueur comprend un composé oxadiazole, de préférence un Pléconaril ou un dérivé de Pléconaril.

11. Utilisation selon l'une quelconque des revendications 7 à 10, dans laquelle le marqueur comprend un groupe rapporteur, de préférence dans laquelle une propriété du groupe rapporteur est altérée quand le marqueur se lie à l'analyte, et/ou de préférence, dans laquelle le groupe rapporteur comprend un groupe fluorescent.

12. Utilisation selon l'une quelconque des revendications 7 à 11, dans laquelle le signal est généré en utilisant une balise moléculaire.

13. Méthode de détection d'un analyte dans un échantillon, ladite méthode comprenant :
(a) la mise en contact de l'échantillon avec un marqueur capable de se lier à l'analyte ;
(b) la détection de la présence et/ou de la quantité du marqueur lié à l'analyte et
(c) la détection ainsi de la présence et/ou de la quantité de l'analyte dans l'échantillon ;
dans laquelle l'analyte comprend une unité de protéine de répétition et dans laquelle une pluralité d'entités de marqueur est capable de se lier à une entité d'analyte unique, de sorte qu'un signal obtenu pour un analyte provenant d'une pluralité de marqueurs soit plus fort que le signal obtenu pour un analyte avec un marqueur unique, et dans laquelle le marqueur est un inhibiteur de fonction capside.

14. Méthode selon la revendication 13, dans laquelle l'analyte comprend une unité de protéine de répétition d'un virus choisi parmi les adéno-virus, les virus de l'herpès, les rotavirus, les réovirus, les picornavirus, les papillomavirus, les polyomavirus, les parvovirus, les parvovirus, les virus de la grippe, les flavivirus et les rétrovirus, de préférence dans laquelle le virus est choisi parmi le HCV, HIV, ou le virus de l'herpes simplex, le rhinovirus humain, la grippe ou un pathogène viral d'une espèce non-humaine.

15. Méthode selon la revendication 13 ou 14, ladite méthode étant une méthode comme ultérieurement définie dans les revendications 9 à 12.
